# EUROPÄISCHE PATENTSCHRIFT

(11) **EP 2 244 118 B1**
(45) Veröffentlichungstag und Bekanntmachung des Hinweises auf die Patenterteilung: **08.08.2012**
(21) Anmeldenummer: 09170849.5
(22) Anmeldetag: 21.09.2009
(51) Int. Cl.: G02B 21/00, G02B 7/02, A61B 3/13

(54) **Vorsatzhalterung für Operationsmikroskop**
Attachment holder for operation microscope
Fixation d'adaptateur pour microscope d'opération

(30) Priorität: 20.04.2009 DE 102009018114
(43) Veröffentlichungstag der Anmeldung: 27.10.2010
(73) Patentinhaber: Dieter Mann GmbH, 63814 Mainaschaff (DE)
(72) Erfinder: Mann, Dieter, 63839, Kleinwallstadt (DE)
(74) Vertreter: Prüfer & Partner GbR European Patent Attorneys

(56) Entgegenhaltungen:
- EP-A2- 1 450 194
- EP-A2- 1 889 567
- EP-A2- 2 096 481
- DE-A1- 10 336 893
- DE-C- 814 798
- US-A1- 2006 232 856

## Beschreibung

Die Erfindung betrifft ein Mikroskop, das eine kontaktfreie Weitwinkelbeobachtung während eines augenchirurgischen Eingriffs erlaubt oder die Durchführung eines Eingriffs unter Zuhilfenahme eines auf das Auge aufgesetzten Kontaktglases gestattet. Speziell bezieht sich die Erfindung auf ein bei einem augenchirurgischen Eingriff verwendbares Operationsmikroskop, das zumindest zeitweilig die Beobachtung des Augenhintergrunds (Fundus) gestattet.

US 2006/0232856 A1 beschreibt ein Operationsmikroskop mit einem optischen Beleuchtungssystem, mit dem Beleuchtungslicht in ein zu operierendes Auge gebracht wird. Das Mikroskop weist einen Objektivtubus mit einem darin angeordneten Objektiv auf sowie eine Vorsatzlinse, die zwischen dem Objektiv und dem Auge angeordnet ist, zum Sammeln des Beleuchtungslichts und Führen des gesammelten Lichts in den vorderen Augenabschnitt zum Beleuchten des Augeninneren. Dabei ist die Vorsatzlinse in einer Haltevorrichtung für den Objektivtubus angebracht, bezüglich welcher sie mittels eines Linearmotors positionierbar ist.

Die europäische Patentanmeldung EP 1 889 567 A2 beschreibt ein Ophthalmoskopievorsatzmodul für ein Operationsmikroskop. Das Vorsatzmodul weist eine Halteeinrichtung für eine Ophthalmoskopierlupe auf. Weiterhin ist das Vorsatzmodul mit einer Halteeinrichtung an einer Aufnahme für ein fokussierbares Mikroskop-Hauptobjektivsystem angeordnet.

Die europäische Patentanmeldung EP 1 450 194 A2 beschreibt ein Operationsmikroskop mit einer Vorsatzlinse. Eine Vorsatzlinsenhalterung ist an der Seitenoberfläche des Mikroskop-Hauptkörpers befestigt.

DE 103 36 893 A1 beschreibt eine abnehmbare optische Vorrichtung zur lösbaren Befestigung an einem zur kontaktfreien Beobachtung eines Auges geeigneten Mikroskops. Eine Linse ist zwischen dem Objektiv des Mikroskops und dem Auge in der optischen Achse des Mikroskops anordenbar. Mittels einer Antriebseinrichtung, mit der die Linse entlang der optischen Achse verstellt werden kann, ist die Linse an dem Objektivgehäuse befestigt.

Bei Operationen im Hinterabschnitt des Auges ist insbesondere eine Weitwinkelbeobachtung erforderlich. Im Stand der Technik ist es bekannt, eine Weitwinkelbeobachtung dadurch vorzunehmen, dass eine Linse zwischen das Objektiv des Mikroskops und das Auge gesetzt wird. Beispielsweise kann eine Kontaktlinse bzw. ein Kontaktglas auf das Auge aufgesetzt werden. Der Nachteil der Verwendung solch einer Kontaktlinse ist jedoch, dass der Bildausschnitt auf maximal 68° begrenzt ist.

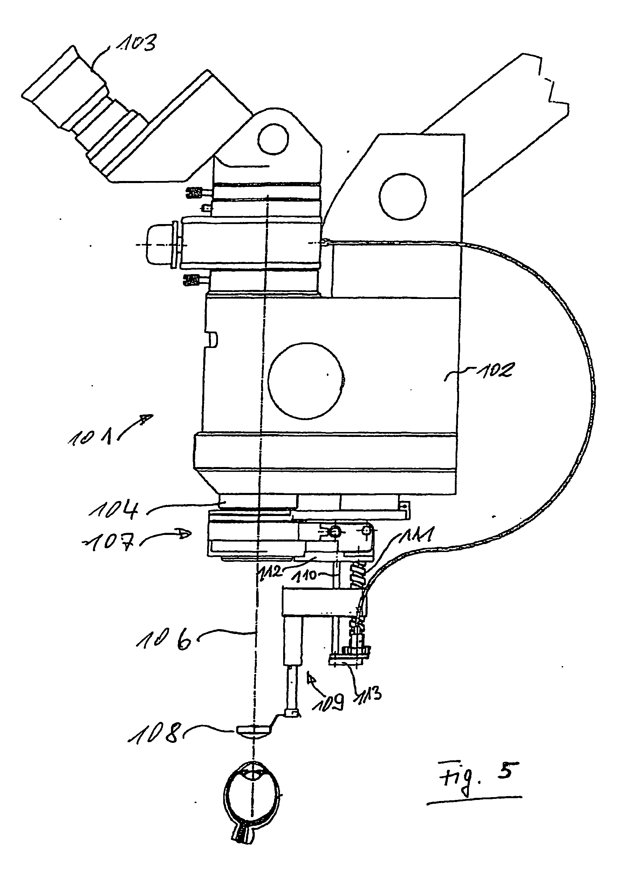

EP 1 199 591 A1 schlägt zur Vermeidung dieses Nachteils vor, zwischen Objektiv und Auge eine elektromotorisch verstellbare Optik vorzusehen, die nicht unmittelbar in Kontakt mit dem Auge ist. Bei dieser Optik gestattet ein elektromotorischer Antrieb ein Nachfokussieren, so dass der Operateur den Eingriff nicht unterbrechen muss. Fig. 5 zeigt die entsprechende Anordnung.

In Fig. 5 weist ein Mikroskop 101 ein Gehäuse 102 mit einem Okular 103 und einem Objektiv 104 auf. An dem Objektiv ist eine Halterung 107 angebracht, die in Richtung der optischen Achse 106 relativ zu dem Objektiv 104 unbeweglich ist und zur Fixierung einer Vorsatzoptik 108 an dem Objektiv 104 dient. Die Optik 108 besteht aus einer Vorsatzlinse, die an einem Haltearm 109 angebracht ist, der an der Halterung 107 entlang der optischen Achse 106 beweglich ist. Dabei sind an der Halterung 107 ein feststehender Stab 110 und eine Gewindespindel 111 angebracht, die sich parallel zu der optischen Achse erstrecken. Der feststehende Stab 110 und die Gewindespindel 111 sind einerseits an einer an dem Objektiv angebrachten Grundplatte 112 der Halterung 107 und andererseits an einer Brücke 113 gelagert.

Die Anordnung ist so ausgestaltet, dass die Vorsatzoptik 108 in den Strahlengang 106 des Mikroskops 101 einschwenkbar ist und aus dieser Position wieder ausschwenkbar ist. Als Nachteil dieses Operationsmikroskops nach dem Stand der Technik wird es gesehen, dass die Fokussierung nach dem Einschwenken oder Ausschwenken der Vorsatzoptik schwierig ist. Wird beispielsweise die Vorsatzlinse 108 in den Strahlengang gebracht, so muss zur Fokussierung nicht nur der Abstand zwischen der Linse 108 und dem Objektiv 104 verändert werden, sondern zusätzlich muss auch der Abstand zwischen dem Objektiv 104 und dem Auge verändert werden. Zwar können die entsprechenden Linsen per Fußschalter elektromotorisch bewegt werden, so dass der Operateur nicht die im Auge befindlichen Instrumente loslassen muss, jedoch ist die Fokussierung dadurch umständlich, dass die Lage zweier Linsen verändert werden muss, welche nicht unabhängig voneinander den Fokus beeinflussen.

Zusätzlich ergibt sich beim Einschwenken einer Linse in den Strahlengang noch das Problem, dass ein seitenverkehrtes, auf dem Kopf stehendes Bild erzeugt wird, was den Operateur wegen der Bewegungsrichtung der Instrumente im Auge entgegen der Bewegungsrichtung der Hand stört. Zwar wird in EP 1 227 355 B1 vorgeschlagen, zusammen mit der Vorsatzlinse eine Bildumkehreinrichtung in den Strahlengang ein- und auszuschwenken, jedoch bleibt das Problem der komplizierten Fokussierung weiterhin bestehen:
Da der Abstand zwischen der Vorsatzlinse und dem Auge in EP 1 199 591 A1 sich jedes Mal ändert, wenn der Fokus geändert wird, ist eine unabhängige Ausrichtung der Vorsatzlinse notwendig.

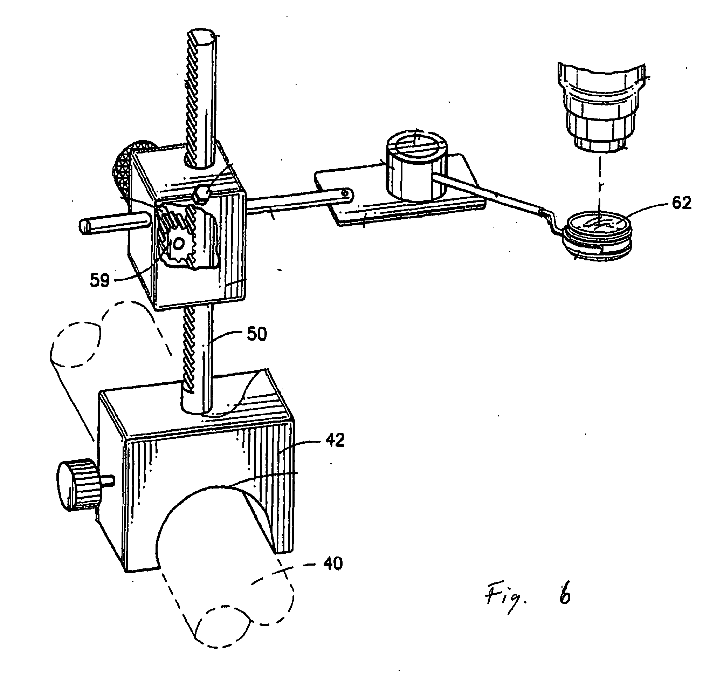

In US 5,793,524 wird daher vorgeschlagen, den Abstand zwischen Vorsatzlinse und Patientenauge zu fixieren. Hierzu befindet sich die Vorsatzlinse an einer ringförmigen Halterung, die um den Kopf des Patienten herum angeordnet ist und am Operationstisch, auf dem der Patient liegt, befestigt ist. In Fig. 6 ist eine derartige Halterung mit der Vorsatzlinse 62 gezeigt. Die Linse ist über eine Zahnstangenvorrichtung 50, 59 in der Höhe verstellbar. In Figur 6 ist auch ein Lagerblock 42 gezeigt, der eine zylinderförmige Ausnehmung zur Befestigung an einer zylinderförmigen Handgelenkstütze 40 für den Operateur aufweist, wie sie in den USA üblicherweise verwendet wird.

Der Nachteil dieser Vorrichtung ist, dass es beispielsweise ausserhalb der USA eher unüblich ist, eine Handgelenkstütze am Operationstisch vorzusehen. Die Vorrichtung macht also einen speziell ausgestalteten Operationstisch notwendig. Ein weiterer Nachteil ist der, dass bei einer Bewegung des Patientenauges einerseits die Lage der Vorsatzlinse korrigiert werden muss und andererseits die Ausrichtung des Mikroskops zum Patientenauge und zur Vorsatzlinse korrigiert werden muss. In solch einem Fall ist eine Unterbrechung des chirurgischen Eingriffs nahezu unvermeidbar.

In ähnlicher Weise ist auch bei Verwendung eines auf das Auge aufzusetzenden Kontaktglases eine Unterbrechung des Eingriffs erforderlich, falls nicht eine zweite Person assistiert, die dann auch das Kontaktglas in Position hält.

Angesichts der oben geschilderten Nachteile des Standes der Technik ist es eine Aufgabe der Erfindung, ein Mikroskop bereitzustellen, das während eines chirurgischen Eingriffs am Auge eine einfache Positionierung eines optischen Vorsatzteils gestattet.

Die Aufgabe wird gelöst durch ein Mikroskop nach Anspruch 1.

Weiterbildungen der Erfindung sind in den Unteransprüchen angegeben.

Weitere Merkmale und Zweckmäßigkeiten der Erfindung ergeben sich aus der Beschreibung von Ausführungsbeispielen auf der Grundlage der beigefügten Figuren. Von diesen zeigen:
Fig. 1a den Gesamtaufbau eines erfindungsgemäßen Operationsmikroskops, bei dem eine Vorsatzlinse zur Weitwinkelbeobachtung in den Strahlengang eingeführt ist,
Fig. 1b eine Seitenansicht einer Haltevorrichtung für die Vorsatzlinse,
Fig. 2 die Konstruktion einer Schwenkvorrichtung,
Fig. 3 das erfindungsgemäße Operationsmikroskop in Kombination mit einem Mitbeobachtermikroskop und einer Spaltleuchte,
Fig. 4 eine alternative Befestigungsmöglichkeit der Haltevorrichtung für die Vorsatzlinse am Haltearm des Operationsmikroskops,
Fig. 5 den Gesamtaufbau eines Operationsmikroskops samt Vorsatzlinse nach dem Stand der Technik und
Fig. 6 eine weitere Möglichkeit der Anbringung einer Vorsatzlinse nach dem Stand der Technik.

### 1. Ausführungsform

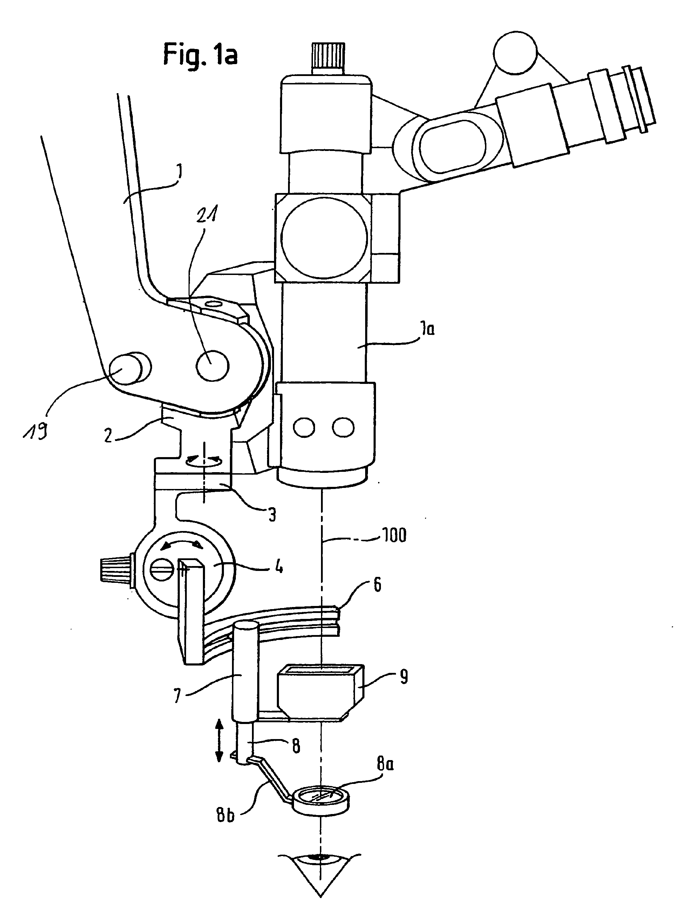

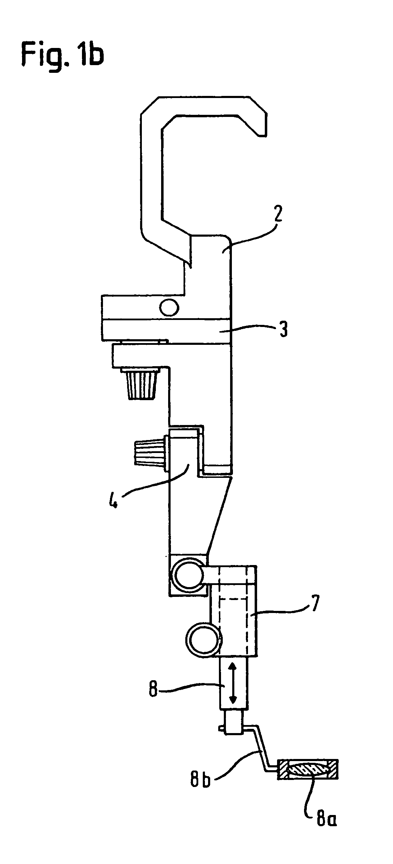

Fig. 1a zeigt eine Ausführungsform des Operationsmikroskops mit Vorsatzlinse für eine Weitwinkelbeobachtung. Ein Tubus 1a mit einem daran befestigten Objektiv und Okular ist an einem Haltearm 1 für das Mikroskop so befestigt, dass eine Bewegung des gesamten Tubus 1a, oder zumindest des Objektivs, gegenüber dem Haltearm 1 entlang der optischen Achse des Objektivs möglich ist. An dem Haltearm ist eine Befestigungsvorrichtung 2 zum Anbringen einer Haltevorrichtung für die Vorsatzlinse an dem Haltearm 1 vorgesehen. Fig. 1b zeigt die Haltevorrichtung für die Vorsatzlinse in Seitenansicht. Die Befestigungsvorrichtung 2 ist dabei beispielhaft als Haken gezeigt. Die Haltevorrichtung setzt sich zusammen aus einer ersten Schwenkvorrichtung 4 zum Verschwenken um eine, beispielsweise horizontale, erste Achse, einer zweiten Schwenkvorrichtung 3 zum Verschwenken um eine zur optischen Achse des Objektivs parallele Achse, einem halbkreisförmigen Träger 6 zum Verdrehen einer Vorsatzlinse 8a um eine zur Linsenebene senkrechte Achse, einer optionalen Bildumkehreinrichtung 9 sowie der eigentlichen Linsenhalterung 7, 8.

In Figur 1a und 1b ist die zweite Schwenkvorrichtung 3 näher an dem Haltearm 1 angeordnet als die übrigen Teile der Haltevorrichtung. Es wäre aber auch möglich, in der Figur die Lage von erster und zweiter Schwenkvorrichtung zu vertauschen.

Insbesondere wäre es auch möglich, die erste Schwenkvorrichtung 4 so auszubilden, dass sie gleichzeitig als Befestigungsvorrichtung 2 zum Anbringen der Haltevorrichtung für die Vorsatzlinse an dem Haltearm 1 dient. In diesem Fall wäre eines der beiden gegeneinander zu verschwenkenden Teile starr mit dem Haltearm 1 verbunden, beispielsweise über eine Schraubverbindung.

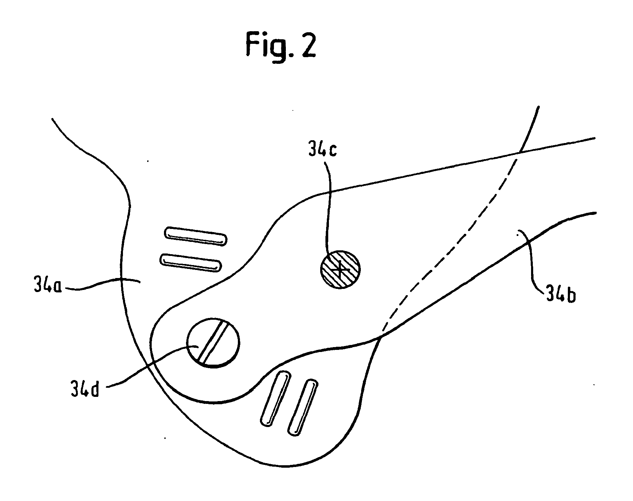

Der Aufbau der Schwenkvorrichtungen 3 und 4 ist beispielhaft in Fig. 2 dargestellt. Sowohl die erste als auch die zweite Schwenkvorrichtung besteht im Wesentlichen aus zwei Teilen 34a und 34b, die miteinander über einen Bolzen 34c so verbunden sind, dass sie gegeneinander verdreht werden können. Das Feststellen der gegenseitigen Lage der gegeneinander verschwenkbaren Teile 34a und 34b geschieht dabei mittels einer Arretiervorrichtung 34d. Beispielsweise kann solch eine Arretiervorrichtung als Arretierschraube ausgebildet sein, welche in ein in dem einen Schwenkteil 34b ausgebildetes Schraubloch geschraubt ist. Das dem Schraubenkopf gegenüberliegende Ende kann beispielsweise halbkugelförmig ausgebildet sein.

Zweckmäßigerweise ist in dem anderen Schwenkteil 34a eine Vertiefung ausgebildet, in welche das Schraubenende eingreift. Zum Ausbilden der Vertiefung kann das Schwenkteil 34a an bestimmten Stellen ausgenommen sein. Alternativ dazu können in dem Schwenkteil 34a Erhebungen ausgebildet sein, zwischen welche das halbkugelförmige Schraubenende eingesenkt werden kann. In der Figur 2 handelt es sich beispielsweise bei den Erhebungen um parallele Stahlstäbe, die zur Hälfte in das andere Teil 34a eingelassen sind.

Der Schraubenkopf der Arretierschraube 34d muss nicht zwingend halbkugelförmig sein, sondern kann in beliebiger Weise ausgebildet sein. Desweiteren ist in Fig. 2 die Arretiervorrichtung lediglich beispielhaft mit einem Hebel gezeigt. Andere Ausgestaltungen des Kopfes der Arretierschraube (Arretiervorrichtung) sind ebenso möglich. Insbesondere ist es möglich, den Schraubenkopf mit einer sterilisierbaren Kappe zu versehen.

Der halbkreisförmige Träger 6 ist als Schiene ausgebildet, auf der eine Linsenträgeraufnahme 7 als Teil der eigentlichen Linsenhalterung entlang gleiten kann. Dabei ist es unerheblich, auf welche Weise die Schiene ausgebildet ist. Insbesondere kann zum Bewegen der Linsenträgeraufnahme 7 auf dem halbkreisförmigen Träger 6 ein Vorsprung der Linsenträgeraufnahme 7 in eine Vertiefung auf dem halbkreisförmigen Träger 6 eingreifen, oder aber ein Vorsprung auf dem halbkreisförmigen Träger 6 greift in eine Vertiefung in der Linsenträgeraufnahme 7 ein.

Der eigentliche Linsenträger 8, der in der Abbildung stabförmig bzw. zylinderförmig ausgebildet ist, ist vertikal, also in Richtung der Zylinderachse, falls er zylinderförmig ist, dergestalt beweglich, dass er in eine, beispielsweise zylinderförmige, Ausnehmung am unteren Ende der Linsenträgeraufnahme 7 eindringt. Dadurch kann der Linsenträger 8 durch Anheben vertikal verstellt werden. Insbesondere findet keine Arretierung in einer angehobenen Position statt. Desweiteren ist durch das Gleiten des oberen Endes des Linsenträgers 8 in dem unteren Ende der Linsenträgeraufnahme 7 kein großer Widerstand zu überwinden, um die Vorsatzlinse samt Linsenträger anzuheben. Dadurch wird verhindert, dass das Patientenauge bei fehlerhafter Positionierung des Operationsmikroskops Schaden nimmt.

Die Vorsatzlinse 8a ist in einem Ring montiert, welcher über einen Tragesteg 8b mit dem Linsenträger 8 verbunden ist. Beispielsweise greift der Tragesteg 8b in einen horizontalen Schlitz am unteren Ende des Linsenträgers 8 ein.

Eine Justage der Vorsatzlinse 8a zum Einbringen derselben in den Strahlengang 100 geschieht innerhalb einer zur optischen Achse des Objektivs senkrechten Ebene über ein Verschwenken der Haltevorrichtung für die Vorsatzlinse 4, 6, 7, 8, 8b gegenüber dem Haltearm 1 mittels der zweiten Schwenkvorrichtung 3. Durch Verfahren der Linsenträgeraufnahme 7 in der Schiene des halbkreisförmigen Trägers 6 können zusätzlich die Linsenträgeraufnahme 7, der Linsenträger 8, der Tragesteg 8b und die Vorsatzlinse 8a bezüglich der Achse 100 des Strahlengangs gedreht werden. Dies ist erforderlich zum Einstellen des halbkreisförmigen Trägers 6, der Linsenträgeraufnahme 7 und des Linsenträgers 8 relativ zur Lidspalte. Eine solche Verstellmöglichkeit ist beispielsweise beim Wechseln von einem Auge auf das andere notwendig oder aber zum Vermeiden eines Kontaktes mit einer Lidsperre.

Wird die Vorsatzlinse 8a nicht benötigt, so kann sie mittels der ersten Schwenkvorrichtung 4 mitsamt der halbkreisförmigen Schiene 6, der Linsenträgeraufnahme 7, dem Linsenträger 8, und dem Tragesteg 8b weggeschwenkt werden.

Bei der soeben beschriebenen Vorrichtung ist die Vorsatzlinse über ihre Halterung am Haltearm 1 des Mikroskops befestigt, so dass sie nicht die Positionsveränderung des Tubus 1a entlang der Achse des Beobachtungsstrahlengangs mitmacht, sondern in der einmal eingestellten Position zum Auge verbleibt. Dadurch ist die Scharfstellung vereinfacht, da nur mehr der Mikroskoptubus 1a vertikal bewegt werden muss, wenn ein scharfes Bild einzustellen ist.

Weiterhin gestattet die Anbringung der Vorsatzlinsenhalterung am Haltearm des Mikroskops die fixe Einstellung der Lage der Vorsatzlinse 8a in einer Ebene senkrecht zum Beobachtungsstrahlengang 100 bzw. senkrecht zur optischen Achse des Objektivs. Dadurch ist das Wiederauffinden des Operationsgebiets bei Bewegungen des Patienten erleichtert. Es müssen nicht Vorsatzlinse und Mikroskop unabhängig voneinander auf die neue Position des Patienten ausgerichtet werden, sondern es können beide gemeinsam ausgerichtet werden, indem das Mikroskop als Ganzes neu ausgerichtet wird.

Da beim Einschwenken der Vorsatzlinse 8a in den Strahlengang des Mikroskops das Bild auf den Kopf gestellt wird und seitenverkehrt wird, ist es notwendig, durch das Einbringen einer Bildumkehreinrichtung, beispielsweise eines Schmidtschen Prismas, das Bild um 180° zu drehen. Wie in Fig. 1 gezeigt, kann solch eine Bildumkehreinrichtung 9 beispielsweise an der Linsenträgeraufnahme 7 angebracht sein, so dass sie gemeinsam mit der Vorsatzlinse 8a in den Strahlengang eingeschwenkt werden kann. Alternativ kann solch eine Bildumkehreinrichtung auch am Tubus 1a angebracht sein und mittels Betätigung per Fußschalter in den Strahlengang verfahren werden. Die Anbringung am Tubus hat den Nachteil, dass dies, infolge der Bauhöhe der Bildumkehreinrichtung, eine Vergrößerung der Bauhöhe des Mikroskops und damit eine, bezogen auf das Patientenauge, höher gelegene Einblickposition bewirkt. Der dadurch vergrößerte Abstand zum Auge kann von manchem Operateur als nachteilig empfunden werden.

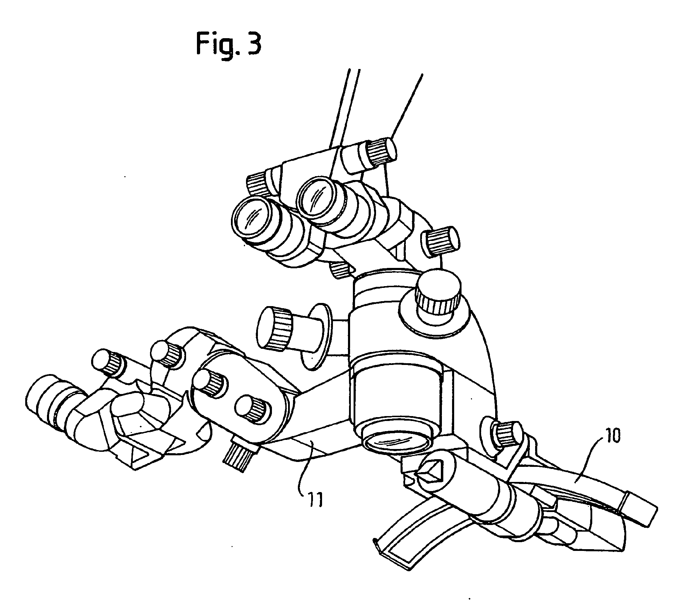

Erfindungsgemäß ist eine Haltevorrichtung für die Vorsatzlinse nicht am Objektiv angebracht. Hierdurch verbleibt im Bereich des Objektivs Raum für Zusatzinstrumente. Fig. 3, in der aus Gründen der besseren Darstellung die erfindungsgemäße Haltevorrichtung nicht gezeigt ist, zeigt ein Mitbeobachtermikroskop 11 sowie eine Spaltleuchte 10 als Beispiele für Zusatzinstrumente. Durch den freien Raum rund um das Objektiv herum ist der Wechsel der Einstellung eines Mitbeobachtermikroskops 11 vom rechten auf das linke Patientenauge und umgekehrt nicht behindert. Des Weiteren ist auch genügend Platz für die Anbringung einer motorischen Spaltleuchte 10 vorhanden. Die gemeinsame Verwendung der Weitwinkel-Vorsatzlinse 8a mit einer Faserspaltleuchte 10 ist von besonderem Vorteil bei der Untersuchung von Kleinkindern (Frühgeborenen).

Die Befestigungsvorrichtung 2 zum Anbringen der Vorsatzlinsenhalterung (3, 4, 6, 7, 8, 8a, 8b) am Haltearm 1 des Mikroskops kann auf verschiedene Art und Weise realisiert werden. Beispielsweise kann diese mittels einer Schwalbenschwanzführung realisiert sein oder aber aus dem in Fig. 1b gezeigten Haken 2 bestehen, der an der Verbindungsachse von Mikroskophaltearm 1 und Mikroskop angebracht ist.

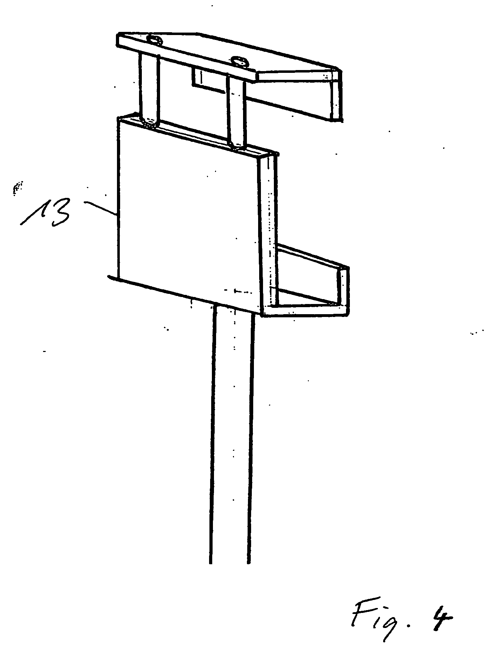

Fig. 4 zeigt noch eine andere Möglichkeit der Befestigung, bei der an der Haltevorrichtung für die Vorsatzlinse ein Schuh 13 ausgebildet ist, welcher den Haltearm 1 umgreift.

Von besonderem Vorteil ist es, wenn die Befestigungsvorrichtung 2 so an dem Haltearm 1 angebracht ist, dass sie gegenüber diesem verkippbar ist. In der Figur 1a ist dies dadurch dargestellt, dass eine Verkippungsachse 21 gezeigt ist, um welche die Vorsatzlinsenhalterung (3, 4, 6, 7, 8, 8a, 8b) zusammen mit dem Mikroskop einschließlich des Tubus 1a verkippen kann. Die Verkippung wird dabei mittels des gezeigten Betätigungsknopfes 19 eingestellt.

Zu beachten ist dabei, dass, obwohl der Tubus 1a bezüglich der Befestigungsvorrichtung 2 höhenverfahrbar ist, der Tubus 1a und die Befestigungsvorrichtung 2 gemeinsam gegenüber dem Haltearm 1 verkippt werden können. Hierdurch ist eine Neuausrichtung des Operationsmikroskops bei Bewegungen des Patienten unter Beibehaltung des Abstandes zwischen Vorsatzlinse und Objektiv besonders erleichtert: Es wird noch ein weiterer Freiheitsgrad der Bewegung für das Operationsmikroskop bereitgestellt.

Natürlich kann die soeben beschriebene Verkippungsmöglichkeit auch mittels der ersten Schwenkvorrichtung 4, die dann gleichzeitig als Befestigungsvorrichtung 2 zur Anbringung am Haltearm 1 dient, bewerkstelligt werden, wie es bereits oben beschrieben wurde.

Obwohl vorstehend stets von einer Vorsatzlinse die Rede war, so ist die Erfindung natürlich auch auf anders gestaltete Vorsatzoptiken, beispielsweise Linsensysteme, anwendbar.

Es ist möglich, eine erste Feineinstellvorrichtung vorzusehen zur Feinpositionierung der Vorsatzlinse 8a auf der optischen Achse des Objektivs.

Weiterhin ist es möglich, eine zusätzliche zweite Feineinstellvorrichtung, beispielsweise einen Kreuztisch, vorzusehen zur Feineinstellung der Lage der Vorsatzlinse 8a in einer Ebene senkrecht zur optischen Achse des Objektivs.

Die erste und zweite Feineinstellvorrichtung können beispielsweise dadurch realisiert werden, dass an der ersten und zweiten Schwenkvorrichtung 3, 4 jeweils eine Exzenterschraube vorgesehen wird, die in die Vertiefung eingreift, in welche auch die Arretiervorrichtung 34d eingreift. Eine Feineinstellung ist dann durch leichtes Verdrehen der Exzenterschraube möglich.

Bevorzugt sollten alle Bestandteile der Vorsatzlinsenhalterung, mit Ausnahme der Bildumkehreinrichtung 9 und der Befestigungseinrichtung 2, dampfsterilisierbar sein.

### Zweite Ausführungsform

Eine zweite Ausführungsform der Erfindung unterscheidet sich von der ersten Ausführungsform lediglich darin, dass es sich bei der in der ersten Ausführungsform erwähnten Vorsatzlinse nun um eine auf die Hornhaut des Auges aufzusetzende Linse oder ein auf die Hornhaut des Auges aufzusetzendes Kontaktglas 8a', welches mehrere Linsen enthalten kann, handelt. Eine derartige Linse oder ein derartiges Kontaktglas, im Folgenden ist der Einfachheit halber, ohne dass es beabsichtigt ist, die Erfindung hierauf einzuschränken, nur von einem Kontaktglas die Rede, sind ebenfalls über eine Vorsatzlinsenhalterung 3, 4, 6, 7, 8, 8b und die Befestigungsvorrichtung 2 am Haltearm 1 des Mikroskops angebracht. Die Handhabung eines derartig gehalterten Kontaktglases geschieht folgendermaßen:

Während eines operativen Eingriffs kann das Kontaktglas mittels der ersten Schwenkvorrichtung 4 und/oder der zweiten Schwenkvorrichtung 3 in den Beobachtungsstrahlengang des Mikroskops eingeschwenkt werden. Hierbei ist natürlich eine Verletzung des Auges zu vermeiden. Diesbezüglich ist es von Vorteil, dass, wie bei der ersten Ausführungsform beschrieben wurde, der Linsenträger 8 in der Linsenträgeraufnahme 7 vertikal frei beweglich ist.

Zum Aufsetzen des Kontaktglases wird zunächst der Linsenträger 8 bis zu seiner höchsten Position in der Linsenträgeraufnahme 7 angehoben. In dieser Stellung wird das Kontaktglas 8a', beispielsweise über einen Haltering und einen Tragesteg 8b, am Linsenträger 8 angebracht. Hiernach wird das Kontaktglas 8a' in den Beobachtungsstrahlengang geschwenkt und der Linsenträger 8, der Tragesteg 8b und das Kontaktglas 8a' werden vorsichtig abgesenkt. Am Ende lastet das Kontaktglas mit seinem Eigengewicht und dem Gewicht des Halterings, des eventuell vorhandenen Tragestegs und des Linsenträgers auf dem Auge, auf dem sich zweckmäßigerweise ein steriles Kontaktgel befindet.

In einer Abwandlung der vorliegenden Ausführungsform kann eine Arretiermöglichkeit für den Linsenträger 8 in seiner höchsten Position in der Linsenträgeraufnahme 7, also der Position, in der er den größten Abstand zum Patientenauge hat, vorgesehen werden. Die Arretierung kann dabei beispielsweise über einen Magnetkontakt zwischen Linsenträger und Boden der Ausnehmung am unteren Ende der Linsenträgeraufnahme 7 geschehen. Damit muss der Linsenträger 8 während der Anbringung des Kontaktglases 8a' an ihm nicht aktiv in seiner höchsten Position gehalten werden, wodurch der Bewegungsablauf für den Bediener einfacher ist. Wenn dann das Kontaktglas 8a' sich im Beobachtungsstrahlengang befindet, wird die Arretierung des Linsenträgers 8 gelöst, so dass das Kontaktglas auf das Patientenauge absinken kann.

Bei einer magnetischen Arretierung könnte die Arretierung beispielsweise unter Zuhilfenahme eines Elektromagneten aktiviert und gelöst werden. Es sind aber natürlich auch mechanische Mittel, wie z.B Rasten, zum Bewerkstelligen einer Arretierung möglich, die ergänzend zu einem Magnetkontakt oder aber anstelle eines Magnetkontaktes verwendet werden können.

Mit der Erfindung ist es somit möglich, das Kontaktglas kontrolliert auf das Auge aufzusetzen und in dieser Position zu fixieren. Das Einschwenken des Kontaktglases in den Beobachtungsstrahlengang und die Höheneinstellung des Kontaktglases können dabei infolge der einfachen und gut kontrollierbaren Handhabung von dem Operateur selbst bewerkstelligt werden, ohne dass es der Unterstützung durch einen Assistenten bedarf. Insbesondere macht es die freie Beweglichkeit des Linsenträgers 8 in der Linsenträgeraufnahme 7 dem Operateur möglich, das Auge bei aufgesetztem Kontaktglas relativ zum Kontaktglas zu bewegen.

Ansonsten sind alle für die erste Ausführungsform beschriebenen Abwandlungen und Ausgestaltungen in gleicher Weise auf die zweite Ausführungsform anwendbar.

Obwohl in der obigen Beschreibung lediglich von Vorsatzlinsen, Vorsatzlinsensystemen oder Kontaktgläsern die Rede war, ist es natürlich möglich, jedwedes optische Bauteil mittels der beschriebenen Vorsatzlinsenhalterung 3, 4, 6, 7, 8, 8b und der Befestigungsvorrichtung 2 am Haltearm 1 des Mikroskops zu befestigen. In den nachfolgenden Ansprüchen ist daher allgemein von einem "optischen Vorsatzteil" die Rede.

## Patentansprüche

1. Operationsmikroskop zur Durchführung eines augenchirurgischen Eingriffs mit:
einem Tubus (1a) mit einem Objektiv und einem Okular, einem Haltearm (1), an dem der Tubus (1a) derart befestigt ist, dass eine Bewegung des Objektivs gegenüber dem Haltearm (1) entlang der optischen Achse des Objektivs möglich ist,
einem optischen Vorsatzteil (8a) zwischen Objektiv und Auge,
einer Haltevorrichtung für das optische Vorsatzteil (8a),
einer Befestigungsvorrichtung (2) mittels derer die Haltevorrichtung für das optische Vorsatzteil am Haltearm (1) befestigt ist,
einer Dreheinrichtung (6, 7) zum Verdrehen des optischen Vorsatzteils (8a) um die optische Achse des Objektivs oder eine zu dieser parallele Achse,
**dadurch gekennzeichnet dass**
die Dreheinrichtung (6, 7) einen halbkreisförmigen Träger (6) aufweist, der als Schiene ausgebildet ist.

2. Mikroskop nach Anspruch 1, das weiterhin eine erste Schwenkvorrichtung (4) zum Verschwenken des optischen Vorsatzteils (8a) um eine Achse aufweist.

3. Mikroskop nach einem der Ansprüche 1 bis 2, bei dem die Befestigungsvorrichtung (2) dergestalt am Haltearm 1 befestigt ist, dass sie zusammen mit dem Tubus (1a) und der Haltevorrichtung für das optische Vorsatzteil gegenüber dem Haltearm um eine Achse (21) verkippbar ist.

4. Mikroskop nach Anspruch 2, bei dem die erste Schwenkvorrichtung so angeordnet ist, dass ein gemeinsames Verschwenken von Dreheinrichtung (6, 7) und optischem Vorsatzteil (8a) möglich ist.

5. Mikroskop nach einem der Ansprüche 1 bis 4, das weiterhin eine zweite Schwenkvorrichtung (3) zum Verschwenken des optischen Vorsatzteils (8a) um eine zur optischen Achse des Objektivs parallele Achse aufweist.

6. Mikroskop nach Anspruch 5, bei dem die zweite Schwenkvorrichtung so angeordnet ist, dass ein gemeinsames Verschwenken von erster Schwenkvorrichtung (4), Dreheinrichtung (6, 7) und optischem Vorsatzteil (8a) möglich ist.

7. Mikroskop nach einem der Ansprüche 1 bis 6, das weiterhin eine Bildumkehreinrichtung (9) aufweist, die zwischen Objektiv und optischem Vorsatzteil (8a) angeordnet ist.

8. Mikroskop nach Anspruch 7, bei dem eine erste Schwenkvorrichtung so angeordnet ist, dass mittels der ersten Schwenkvorrichtung (4) ein gemeinsames Verschwenken der Bildumkehreinrichtung (9) und des optischen Vorsatzteils (8a) möglich ist.

9. Mikroskop nach Anspruch 7, bei dem eine zweite Schwenkvorrichtung so angeordnet ist, dass mittels der zweiten Schwenkvorrichtung (3) ein gemeinsames Verschwenken der Bildumkehreinrichtung (9) und des optischen Vorsatzteils (8a) möglich ist.

10. Mikroskop nach Anspruch 2, 4 oder 8, bei dem die erste Schwenkvorrichtung (4) gleichzeitig als Befestigungsvorrichtung (2) zur Befestigung der Haltevorrichtung für das optische Vorsatzteil am Haltearm (1) dient.

11. Mikroskop nach einem der Ansprüche 1 bis 10, bei dem zusätzlich eine Faserspaltleuchte angebracht ist, so dass eine Beleuchtung mit der Faserspaltleuchte (10) durch das optische Vorsatzteil (8a) hindurch möglich ist.

12. Mikroskop nach einem der Ansprüche 1 bis 11, bei dem es sich bei dem optischen Vorsatzteil um eine Linse oder ein Linsensystem handelt, wodurch eine kontaktfreie Weitwinkelbeobachtung des Augenhintergrundes ermöglicht wird.

13. Mikroskop nach einem der Ansprüche 1 bis 11, bei dem es sich bei dem optischen Vorsatzteil um ein auf das Auge aufzusetzendes Kontaktglas handelt.

14. Mikroskop nach einem der Ansprüche 1 bis 13, bei dem weiterhin das optische Vorsatzteil an einem Linsenträger (8) angebracht ist, welcher in einer Linsenträgeraufnahme (7) angeordnet ist, aus der er nach unten herausragt,
wobei die Linsenträgeraufnahme Bestandteil der Haltevorrichtung für das Vorsatzteil ist und
der Linsenträger (8) innerhalb einer im Vorhinein festgelegten Distanz innerhalb der Linsenträgeraufnahme (7) frei, d.h. ohne einen die Bewegung hindernden Widerstand, bewegbar ist.

## Claims

1. Surgical microscope for making an ophthalmo-surgical intervention having:
a tube (1a) having an objective and an ocular,
a holding arm (1), at which the tube (1a) is fastened such that a movement of the objective against the holding arm (1) along the optical axis of the objective is possible,
an optical accessory part (8a) between the objective and the eye,
a holding device for the optical accessory part (8a),
an attachment device (2), by means of which the holding device for the optical accessory part is fixed at the holding arm (1),
a rotation device (6, 7) for rotating the optical accessory part (8a) around the optical axis of the objective or an axis parallel to it,
**characterized in that**
said rotation device (6, 7) comprises a semi-circular support, which is designed as guide rail

2. The microscope according to claim 1, which furthermore comprises a first swivelling device (4) for swivelling the optical accessory part (8a) around an axis.

3. The microscope according to one of claims 1 to 2, in which the attachment device (2) is mounted on the holding arm (1) such that it can be tilted relative to the holding arm around an axis (21) together with the tube (1a) and the holding device for the optical accessory part.

4. The microscope according to claim 2, in which the first swivelling device is arranged such that a common swivelling of the rotation device (6, 7) and the optical accessory part (8a) is possible.

5. The microscope according to one of claims 1 to 4, which furthermore comprises a second swivelling device (3) for swivelling the optical accessory part (8a) around an axis that is parallel to the optical axis of the objective.

6. The microscope according to claim 5, in which the second swivelling device is arranged such that a common swivelling of the first swivelling device (4), the rotation device (6, 7) and the optical accessory part (8a) is possible.

7. The microscope according to one of claims 1 to 6, which further comprises an image reversal device (9) that is arranged between the objective and the optical accessory part (8a).

8. The microscope according to claim 7, in which a first swivelling device is arranged such that by means of the first swivelling device (4) a common swivelling of the image reversal device (9) and the optical accessory part (8a) is possible.

9. The microscope according to claim 7, in which a second swivelling device is arranged such that by means of the second swivelling device (3) a common swivelling of the image reversal device (9) and the optical accessory part (8a) is possible.

10. The microscope according to claim 2, 4 or 8, in which the first swivelling device (4) at the same time serves as attachment device (2) for attaching the holding device for the optical accessory part at the holding arm (1).

11. The microscope according to one of claims 1 to 10, in which in addition a fibre slit lamp is attached, so that an illumination with the fibre slit lamp (10) through the optical accessory part (8a) is possible.

12. The microscope according to one of claims 1 to 11, wherein said optical accessory part is a lens or a lens system, such that a contactless wide-angle observation of the fundus is made possible.

13. The microscope according to one of claims 1 to 11, wherein said optical accessory part is a contact glass to be put onto the eye.

14. The microscope according to one of claims 1 to 13, in which said optical accessory part is mounted on said lens support (8), which is arranged in said lens support receptacle (7) such that it protrudes from it at the bottom,
wherein said lens support receptacle is a component of the holding device for the optical accessory part and
said lens support (8) is able to move within a predetermined distance in the lens support receptacle freely, which means without any resistance that is impeding the movement.

## Revendications

1. Microscope opératoire utilisé pour les interventions de chirurgie oculaire, comportant :
un tube (1a) avec un objectif et un oculaire,
un bras de fixation (1), auquel le tube (1a) est fixé de telle sorte qu'un mouvement de l'objectif par rapport au bras de fixation (1) est possible le long de l'axe optique de l'objectif,
un élément additionnel optique (8a) entre l'objectif et l'oeil,
un dispositif de support pour l'élément additionnel optique (8a),
un dispositif de fixation (2) par lequel le dispositif de support de l'élément additionnel optique est fixé sur le bras de fixation (1),
un dispositif rotatif (6, 7) pour faire tourner l'élément additionnel optique (8a) autour de l'axe optique de l'objectif ou d'un axe parallèle à celui-ci,
**caractérisé en ce que**
le dispositif rotatif (6, 7) comporte un support (6) semi-sphérique réalisé en forme de rail.

2. Microscope selon la revendication 1, comportant en outre un premier dispositif de pivotement (4) pour faire pivoter l'élément additionnel optique (8a) autour d'un axe.

3. Microscope selon l'une quelconque des revendications 1 à 2, dans lequel le dispositif de fixation (2) est fixé sur le bras de fixation (1) de telle sorte que, conjointement avec le tube (1a) et le dispositif de support pour l'élément additionnel optique, il peut basculer par rapport au bras de fixation autour d'un axe (21).

4. Microscope selon la revendication 2, dans lequel le premier dispositif de pivotement est disposé de telle sorte que le dispositif rotatif (6, 7) et l'élément additionnel optique (8a) peuvent pivoter conjointement.

5. Microscope selon l'une quelconque des revendications 1 à 4, comportant en outre un deuxième dispositif de pivotement (3) pour faire pivoter l'élément additionnel optique (8a) autour d'un axe parallèle à l'axe optique de l'objectif.

6. Microscope selon la revendication 5, dans lequel le deuxième dispositif de pivotement est disposé de telle sorte que le premier dispositif de pivotement (4), le dispositif rotatif (6, 7) et l'élément additionnel optique (8a) peuvent pivoter conjointement.

7. Microscope selon l'une quelconque des revendications 1 à 6, comportant en outre un dispositif d'inversion d'image (9) qui est monté entre l'objectif et l'élément additionnel optique (8a).

8. Microscope selon la revendication 7, dans lequel un premier dispositif de pivotement est disposé de telle sorte qu'un pivotement conjoint du dispositif d'inversion d'image (9) et de l'élément additionnel optique (8a) est possible au moyen du premier dispositif de pivotement (4).

9. Microscope selon la revendication 7, dans lequel un deuxième dispositif de pivotement est disposé de telle sorte qu'un pivotement conjoint du dispositif d'inversion d'image (9) et de l'élément additionnel optique (8a) est possible au moyen du deuxième dispositif de pivotement (3).

10. Microscope selon la revendication 2, 4 ou 8, dans lequel le premier dispositif de pivotement (4) fait en même temps fonction de dispositif de fixation (2) pour fixer sur le bras de fixation (1) le dispositif de support pour l'élément additionnel optique.

11. Microscope selon l'une quelconque des revendications 1 à 10, dans lequel il est prévu, en plus, une lampe à fente en fibres (10), ladite lampe à fente en fibres permettant d'éclairer à travers l'élément additionnel optique (8a).

12. Microscope selon l'une quelconque des revendications 1 à 11, dans lequel l'élément additionnel optique est une lentille ou un système de lentilles, permettant une observation à grand angle sans contact du fond d'oeil.

13. Microscope selon l'une quelconque des revendications 1 à 11, dans lequel l'élément additionnel optique est un verre de contact à poser sur l'oeil.

14. Microscope selon l'une quelconque des revendications 1 à 13, dans lequel, en outre, l'élément additionnel optique est monté sur un porte-lentille (8) qui est disposé dans un logement (7), hors duquel il s'avance vers le bas,
ledit logement faisant partie du dispositif de support pour l'élément additionnel optique, et
le porte-lentille (8) étant librement mobile, c'est-à-dire sans résistance empêchant un mouvement, à l'intérieur d'une distance fixée au préalable à l'intérieur du logement (7).
